# EUROPEAN PATENT SPECIFICATION

(11) **EP 1 596 973 B1**
(45) Date of publication and mention of the grant of the patent: **04.04.2007**
(21) Application number: 04707509.8
(22) Date of filing: 03.02.2004
(51) Int. Cl.: B01F 13/00, A61F 2/46

(54) **ORTHOPAEDIC CEMENT MIXING DEVICE**
VORRICHTUNG ZUM MISCHEN VON ORTHOPÄDIE-ZEMENT
DISPOSITIF DE MELANGE DE CIMENT ORTHOPEDIQUE

(30) Priority: 05.02.2003 GB 0302661
(43) Date of publication of application: 23.11.2005
(73) Proprietor: SUMMIT MEDICAL LIMITED, Bourton on the Water, Gloucestershire GL54 2HQ (GB)
(72) Inventor: FOSTER, David, Oxforshire OX20 1QH (GB)
(74) Representative: Hughes, Andrea Michelle
(86) International application number: PCT/EP2004/000980
(87) International publication number: WO 2004/069396

(56) References cited:
- WO-A-01/56514
- US-A- 4 721 390
- US-A- 5 494 349
- US-A- 5 558 136

## Description

The present invention relates to a device for mixing and delivering orthopaedic bone cement or the like.

Orthopaedic bone cement is used throughout the world to secure hip, knee and other anatomic prostheses in an appropriate anatomical position. The bone cement is produced by thoroughly mixing together two components, usually methylmethacrylate monomer liquid and polymethylmethacrylate powder. The mixing was previously carried out using a simple bowl and spatula. The surgeon then removes the required amount of cement and manipulates it by hand before inserting it into a preformed cavity or applying it to a resected bony surface where the prosthesis is to be positioned. Cement may either be applied by hand or may be put into a syringe and applied thereby.

Several improvements have been made to this mixing arrangement, including providing arrangements for mixing under vacuum, and to improve the mixing efficiency, to result in a homogenous cement material.

Several devices for mixing cement, usually in a vacuum, are presently available and in general use.

Of the available devices, the preferred forms are the 'bowl' type mixers and the 'syringe' mixers.

Bowl type mixers are provided usually in the form of hand-held mixing bowls. The substances to be mixed are placed in the bowl to which a vacuum is applied. The substances are mixed by means of a rotating paddle extending into the bowl, which is rotated manually by means of a handle extending through the lid of the bowl. In some applications, the use of such a mixing bowl, an example of which is disclosed in EP-A-0616552, is favoured. Many surgeons prefer to 'hand pack' the cement. Bowl mixing also tends to be preferred by nurses who are used to the convenience of mixing in this vessel. A bowl is easier to use and it is important that the nurses feel confident, since timing is very crucial and the mixture must be 'right first time'. Many surgeons also tend to prefer bowl mixers. It is crucial that the mixture does not begin to set before it is applied and experienced surgeons can tell, by touch, when the cement is at the right stage for applying to the bone cavity.

These 'bowl' mixers are in widespread use and are very popular. They are easy to use, allowing repeatable consistent mixing, independent of the level of skill of the user, and the concept of mixing used by the bowl is simple and is popular with nurses. The bowl type mixer is very flexible in that it can be used to mix all types of cement and can be used to mix varying quantities of cement. In bowl mixers where a high vacuum is applied, the cement has low porosity and thus high strength.

In the bowl mixer of EP-A-0616552, which has a 'rotating axis', as opposed to the paddle having a fixed axis, the cement is very thoroughly mixed and the chances of 'dead spots' or areas of unmixed cement occurring are very small.

In some applications, it is preferable or even necessary, to apply the mixed cement to the bone or bone cavity by means of a syringe. Indeed, many surgeons prefer syringe-type application to 'hand packing'.

If, for such applications, the cement is initially mixed in a bowl as described above, it must then be transferred to a dispensing syringe. This transfer can be messy and time-consuming and may expose the mixture to more air entrapment. The introduction of air into the cement produces a weak cement, which has obvious disadvantages.

To overcome this problem, mixing devices have been designed which combine a mixing chamber and a syringe. For example, EP-A-0178658 discloses a device for mixing bone cement comprising a mixing container connected to a feed device. A vacuum source is connected to the feed device for mixing the substances under vacuum. This device has proved to be a very efficient mixing and transfer system and eliminates the need to transfer the mixed cement from the mixing bowl to a syringe.

US 4,758,096 and US 3,606,094 also disclose bone cement mixers in which the cement is mixed in the dispensing vessel itself. In the first of these patents, the mixing is effected manually by means of a 'masher' plate-type agitator. The masher plate is attached to a shaft attached to a handle. The agitator is moveable in the chamber both axially and rotatably to permit mixing of the cement by the user, moving the handle vertically and rotatably. However, such mixing operation is difficult and inefficient and does not guarantee thorough mixing of the cement. Partial strokes of the 'masher' can lead to areas of unmixed powder and the mixing is not consistent, and is reliant on the consistency of the user.

Another problem with the 'masher' type system is that it is difficult to mix standard viscosity cement using this plunger. As the plate is pushed down into the cement, it meets a high resistance, which can result in only a partial mixing stroke being carried out and the cement being compacted at the base of the mixing chamber.

Other, improved mix-in-the-syringe mixers are disclosed in, for example, DE-C-883326 and EP-A-0744991.

Again, these mix-in-the-syringe mixers have become very popular and are in widespread use.

The mix-in-the-syringe mixers are very useful where relatively small quantities of cement are mixed and used. Most mixers on the market are designed to be able to mix up to a 'double' mix of the highest volume cement currently in common use (e.g. Simplex Cement).

Several different types of cement are commonly used in orthopaedic applications. These cements have very different characteristics and volumes, and also have different viscosities. Table 1 below shows three of the most commonly used cement types, showing approximate volumes of dry powder, and the corresponding volume of mixed cement.

### Cement Volumes:

| Cement Type | Dry Powder Volume mm³ | Mixed Cement Volume mm³ |
|---|---|---|
| SIMPLEX (USA) | 302,000 | 135,000 |
| PALACOS | 125,000 | 130,000 |
| CMW | 130,000 | 139,000 |

From the above table, it can be seen that cements such as SIMPLEX have a dry powder volume roughly three times that of other cements, to produce the same volume of mixed cement.

Thus, generally, for mix-in-the-syringe mixers, the mixing chamber must be large enough to receive, say, a double mix of dry powder of the highest volume cement commonly used, even though the actual volume of mixed cement is considerably less. This means that generally the body of the syringe or the mixing chamber is, in fact, much larger than necessary for other types of cement, and requires a longer mixing stroke than would be required for the other low volume cements.

In some cases, the surgeon will wish to prepare an even larger quantity of cement, e.g. a triple mix. Larger amounts of cement are required, for example, in a revision operation, or for certain types of primary hips.

Such larger quantities can generally be mixed in a bowl-type mixer, as described, for example, in EP-A-0616552. However, with the syringe-type mixers, it is generally not possible to merely increase the dimensions of the mixing chamber, to allow more cement to be mixed.

Increasing the height of the mixing chamber to accommodate the larger quantity of cement is not feasible, as this results in a device which is just too big to handle comfortably. The longer the chamber is, the more difficult it becomes to introduce the mixing paddle through the column in a correct alignment and to locate the paddle correctly. This is particularly so when the cement becomes more dense and it becomes extremely difficult to push the paddle down and to cause effective mixing.

Thus, there is a need for an effective and efficient, easy to handle, combined mixer and syringe type orthopaedic cement mixer which is not unnecessarily big but which is capable of mixing large, e.g. triple, mixes of cement, even when the cement used is a large volume cement, such as Simplex.

One mixer which aims to deal with this problem is produced by Stryker (the 'Stryker mixer'), and is described in US patent number 5,558,136 and associated patents. This mixer comprises a funnel section, leading into a cylindrical syringe body mixing chamber. A mixing paddle extends through the funnel section and is rotated by means of a handle in the lid of the funnel section. A large quantity of powder can then be inserted into the device through the funnel. When the monomer is added, and the cement powder and monomer mix, the resulting mixture has a smaller volume which is accommodated within the syringe body. The funnel part may then be removed, leaving a simple syringe body for attachment to a syringe gun and nozzle. However, this device relies on the use of low viscosity cement which, as mixed, falls, under gravity, into the syringe body mixing chamber. It is also essential that the mixing phase is started straight away, as soon as the monomer is added, before the mixture starts to 'dough up'.

A problem is that unmixed powder can be left in the funnel as the cement reduces, which does not fall into the syringe body mixing chamber.

This can result in the unmixed powder falling into the mixed cement as the funnel part is removed, resulting in dry or dead spots and, thus, a brittle cement.

Another problem is that when mixing standard cements or where mixing is not started straight away, the doughing-up cement can stick to the walls of the funnel and the paddle causing high wastage.

EP-A-1257237 discloses an apparatus for mixing orthopaedic cement, comprising a first funnel or bowl shaped introduction chamber, removably attached to one end of a second cylindrical mixing chamber, adapted to form the body of a dispensing syringe; and a mixing mechanism comprising a rotatable shaft extending through the first and second chambers, the shaft having at least one radially extending blade. The profile of the blade in the first chamber tapers inwards, towards the second chamber, and extends into and substantially along the length of the second chamber. This mixer, however, has a rather complex design, resulting in higher production costs, also, mixing, particularly with a fairly viscous cement, can be difficult in the relatively long mixing chamber and the mixing blade needs to be particularly strong. The shape of the device is also not particularly easy to hold during mixing.

US 5,494,349 describes a bone cement mixing device comprising a mixing bowl and a lid.

The present invention aims to provide an efficient and effective mixing system which can also mix relatively large quantities of cement.

As mentioned above, the preferred cements are Simplex and PalacosR. Although both of these cements are in widespread use, it tends to be that the favoured cement in the US is Simplex, whereas PalacosR tends to be preferred in Europe. PalacosR is a much more viscous cement than Simplex.

In prior art, so-called 'mix and dump' systems such as that disclosed in US patent no. 5,558,136, a vacuum is applied to both the mixing chamber and the syringe, e.g. by means of a vacuum shroud surrounding these parts. Cement is then mixed in the mixing chamber which is, at this stage, separated from the cylindrical body of the syringe by a piston located between the opening at the bottom of the mixing chamber and the top of the syringe body.

When the cement has been thoroughly mixed, a plunger is pushed downwards to release the piston from its position. The piston and the mixed cement then fall, under gravity, into the syringe until the piston is at the bottom of the syringe and all of the mixed cement has fallen into the syringe body. The mixing chamber is then removed and a dispensing nozzle applied in its place to enable the mixed cement to be dispensed by pushing the piston back up the syringe body, e.g. by means of a plunger or dispenser gun.

During mixing, slight increases in the ambient temperature have a great effect on the viscosity of the cement, with only a few degrees increase in temperature causing a marked increase in the cement viscosity. A problem with systems which rely on the piston being held by friction and falling under gravity is that with more viscous cements, e.g. Palacos, generally, or less viscous cements such as Simplex which have become more viscous due to an increase in temperature, the mixed cement does not fall so readily under vacuum into the syringe body. Furthermore, wear, changes in manufacturing tolerances, etc. and loose powder particles or cement can hinder the piston in falling down to the bottom of the syringe. Thus, the 'dumping' stage in which the mixed cement is 'dumped' into the syringe body ready for delivery cannot always be completed effectively.

One aspect of the present invention aims to solve this problem by providing an orthopaedic cement mixing apparatus comprising a mixing chamber and a cylindrical dispensing chamber, the mixing chamber having an outlet opening and the dispensing chamber having an inlet opening, the outlet opening and the inlet opening being arranged so as to allow cement mixed in the mixing chamber to pass into the dispensing chamber; closure means having a first position separating the outlet opening of the mixing chamber and the inlet opening of the dispensing chamber; and means for applying a vacuum to the mixing chamber and to the dispensing chamber; characterised in that the apparatus further comprises switching means for switching the applied vacuum between the mixing chamber and the dispensing chamber.

In this arrangement, in the preferred embodiment, the closure means is a piston which, in its first position, is located at the top of the dispensing chamber which is preferably the cylindrical body of a dispensing syringe. The cement components are put into the mixing chamber and the vacuum switch is switched such that vacuum is applied to the mixing chamber, whilst the syringe body is at atmospheric pressure.

Thus, the cement is thoroughly mixed under vacuum which avoids introduction of air which causes a brittle cement. At the same time, the fact that there is a pressure differential holds the closure or piston in place to avoid cement falling into the dispenser body.

Once the cement is thoroughly mixed it needs to be 'dumped' into the syringe body. Thus, the vacuum switch is switched such that vacuum is now applied to the syringe body whilst the mixing chamber is now at atmospheric pressure. This pressure differential draws the closure or piston down within the dispensing body to the bottom, and draws the mixed cement with it until all of the cement is located in the dispensing body or syringe. The mixing chamber can then be removed and a nozzle attached in its place to allow the cement to be dispensed by pushing the closure or piston which is now at the bottom of the dispensing body forwards towards the nozzle.

Such a vacuum switching mechanism could be incorporated into any existing 'mix and dump' type mixing mechanism and could be used, for example, to replace the vacuum shroud of the above-described Stryker system as a solution to the problems mentioned above.

The inventors have found that this vacuum switching mechanism incorporated into a mix-and-dump system such as the Stryker system, works well with low viscosity cement such as Simplex, and at relatively low temperatures.

The arrangement is an improvement on the Stryker system where the cement and piston fall under gravity, as discussed above However, although this aspect of the invention solves the problems of a free-falling piston mentioned above, at higher temperatures and/or with higher viscosity cements, the amount of wastage of cement in the mixing system is still relatively high. Furthermore, experience has shown that an eccentric mixing paddle such as in the bowl mixers described in the introduction to this application, e.g. that disclosed in EP-A-0616552, improves the mixing effect.

One solution to the wastage problem, which has been incorporated into commercial embodiments of the Stryker mixer, is to extend the paddle on both sides of the shaft. The paddle rotates with the central shaft, during mixing, and is arranged and positioned so as to continually rotate around the inside of the mixing funnel or chamber during mixing. This has been found to reduce the amount of wastage, since the scraper does continually scrape around the inside wall of the mixing chamber scraping any mixed cement from the walls and depositing it downwards towards the opening between the mixing chamber and the syringe body. The only noticeable wastage is a small amount of cement which adheres to the mixing blades or paddle, and a very small amount adheres to the metal scraper.

Whilst this does provide some solution to the wastage problem, it does not solve the problem of the quality of the mixed cement. As mentioned above, nurses have been found to prefer a bowl-shaped mixer and/or mixers where they can hold the top of the lid while rotating the handle.

Accordingly, in accordance with a further aspect of the invention, there is provided an orthopaedic cement mixing apparatus comprising a mixing chamber and a dispensing chamber, the mixing chamber having an outlet aperture and the dispensing chamber having an inlet aperture, the outlet aperture and the inlet aperture being in cement flow communication, the apparatus further comprising a mixing paddle extending into said mixing chamber and a rotatable handle coupled to said paddle by a gear mechanism arranged such that rotation of said handle causes said paddle to rotate about its own axis and also moves the axis of rotation of the paddle within the chamber whereby the paddle is moved around substantially the entire cement containing region of the interior of the chamber. Again, this particular mixing mechanism, which may be, for example, similar to that described in WO 93/10892, provides mixing quality advantages in any so-called 'mix-and-dump' systems. Particular advantages are provided by the combination of this system with the vacuum switching arrangement of the first aspect of the invention. In the most preferred embodiment, the mixing chamber is a bowl mixer again as described in WO 93/10892 since, as mentioned above, such chambers are easier to handle due to its bowl shape and also because users can hold the lid with one hand and turn the handle with the other. The combination of the first and second aspects of the invention provide a mix-and-dump type system which mixes the cement more thoroughly and provides a better quality of cement and which also has an improved 'dumping' mechanism over the free-fall system described above.

This particular arrangement, with an off-centred mixing system, such as in the bowl mixer described in EP-A-0616552, has proved to provide a much better quality mixed cement generally and, in particular, for Palacos and cement when mixed at higher temperatures, i.e. higher viscosity cement.

Although, with the off-centred mixing arrangement described above, the amount of wastage is relatively small, it was still found to be higher than with the Stryker system provided with the extended paddle as described above. The off-centred paddle of EP-A-0616552 has a paddle which extends radially all the way to the inner surface of the bowl and scrapes cement of the sides and directs it down towards the bottom to be brought into the mixture. However, the main purpose of this feature of the paddle extending out to the sides of the bowl was, rather, to cause unmixed powder to drop down from the sides of the bowl into the cement, and to cut through the cement during mixing to improve the quality of the mix. Such a paddle, due to the fact that the mixing paddle rotates about its own axis as it rotates around the bowl, does not continuously scrape around the inside of the bowl but, rather, touches the inside surface of the bowl and then moves away, continually, leaving a crenulated pattern of cement residue remaining on the inside of the bowl.

In order to resolve this problem, the present invention, according to a third aspect, provides an orthopaedic cement mixing apparatus comprising a mixing chamber, a mixing paddle extending into said chamber and a rotatable handle coupled to said paddle by a gear mechanism arranged such that rotation of said handle causes said paddle to rotate about its own axis and also move the axis of rotation of the paddle within the chamber whereby the paddle is moved around substantially the entire cement containing region of the interior of the chamber; characterised by a scraper element connected to said gear mechanism so as to rotate with the axis of rotation of the paddle in the same or in the opposite direction. The shape of the scraper is preferably such that it extends radially from the gear mechanism to the inside wall of the mixing chamber and extends axially to conform to the shape of the inside wall of the mixing chamber. Thus, as the rotating paddle rotates around the bowl and around its own axis, the scraper rotates around the inside wall of the mixing chamber scraping cement from the inside wall.

This particular paddle and scraper arrangement will find advantages in all types of mixing apparatus such as the hand-held bowl described in WO 93/10892. The paddle and scraper arrangement can be advantageously incorporated into the mixing chamber of any of the above-mentioned mix-and-dump systems.

It has been found that, with the preferred design of the scraper, the scraper not only scrapes cement of the side walls of the mixing chamber, thus reducing wastage, but also contributes to the mixing effect improving the quality of the mixed cement.

Tests found, using such an arrangement, that there was hardly any wastage on the inside of the mixing chamber, with only a small amount of wastage on the main paddle.

In a 'mix-and-dump' system, the cement is first mixed in a mixing chamber and is then 'dumped' down into the cylindrical syringe body ready to be dispensed by means of the syringe.

As described above, in the previous systems, such as that described in US 5,558,136, during mixing, a piston is located and held to seal off the central opening in the bottom of the mixing bowl, where this is attached to the syringe body. Then, once the cement is mixed, the piston is released by means of a plunger, for example, out of its fixed position and then drops down to the bottom of the syringe under gravity and the weight of the cement. In the alternative embodiment using the first aspect of the present invention, the piston does not fall under gravity but, rather, begins to fall as the vacuum is switched from the mixing chamber to the syringe body, the piston then being drawn under vacuum to the bottom of the syringe.

One problem with this arrangement is that the piston was held in place between the mixing chamber and the syringe by friction until the vacuum is applied and then drops down to the bottom of the syringe. This friction arrangement requires very precise dimensions and fitting and can be affected by aging, sterilising processes, etc. which commonly occur in such systems. Thus, because of these effects, the piston might not drop down when needed or, alternatively may drop down when not required, e.g. during transportation.

In accordance with a further aspect, the present invention provides a solution to this problem by providing an orthopaedic cement mixing apparatus comprising a mixing chamber and a dispensing chamber, the mixing chamber having an outlet aperture and the dispensing chamber having an inlet aperture, the inlet aperture and the outlet aperture being in cement flow communication, and further comprising closure means which, in a first position, separates the outlet aperture from the inlet aperture preventing flow of cement from the mixing chamber to the dispensing chamber; characterized in that the closure means is held in its first position by a releasable fastening means. The releasable fastening means may be, for example, a removable pin which passed through the wall of the dispensing chamber and into the closure, e.g. a piston as described above. When the pin is removed by the user, the closure, e.g. a piston, is then free to fall, either under gravity or under the pressure differential as described in relation to the second embodiment of the present invention, to the bottom of the dispensing chamber. In another embodiment, the releasable fastening means is provided by an O-ring which is dimensioned such that it provides a friction grip between the closure wall and the inner wall of the dispensing chamber. With this arrangement, the closure, which is preferably a piston which falls to the bottom of the dispensing chamber and is used then to dispense the mixed cement, is fairly lightweight and not particularly strong. The problem, therefore, is that when the piston is used in the reversed direction to push the mixed cement out of the dispensing chamber, it is not strong enough to push the relatively thick cement forwards. Thus, the 0-ring, in the preferred embodiment is adapted such that when it reaches the bottom of the dispensing chamber, it engages with a further component, e. g. a collar or a further piston, to force the O-ring radially outwards to form a tighter seal with the inner wall of the syringe.

Again, this particular closure arrangement can be used with any of the known mix-and-dump systems discussed above. However, it is preferably used in combination with systems wherein the mixing chamber and the dispensing chamber are simultaneously, during the whole mixing and dumping process, under a vacuum, rather than in systems where the vacuum is switched between the chambers, because the O-ring, in the first position, does not provide an air-tight seal and there is, then, a risk of air leaking into the cement.

In the preferred embodiment of the O-ring feature, the closure or piston is provided with a light seal or an O-ring sufficient to hold the piston in place between the mixing chamber and the syringe. When vacuum is applied, after mixing, the piston then falls down to the bottom of the syringe where a further component is provided which engages with the piston and the O-ring to press out the O-ring to form a tighter seal with the inner wall of the syringe so that when the piston and this additional component are then pushed upwards by the delivery gun to dispense the cement, a tighter seal is provided.

Whilst this arrangement was an improvement on existing systems, it was found, however, that this seal did not hold in place securely enough at the top of the syringe during transportation.

Further ideas were then considered as to how best to hold the piston in place between the mixing chamber and the syringe during mixing. Several ideas were suggested such as locking pins, locking tabs, etc. None of these were, however, considered to be ideal.

Further development led to the idea of not having the piston located at the top between the mixing chamber and the syringe but, rather, merely to have the piston located at the bottom of the syringe for dispensing the mixed cement (as in the mixer described in WO 95/22402) In that case the piston can be a solid, robust piston sufficient to force out mixed cement through a nozzle, but which does not need to be light enough to fall under gravity or to be drawn down under a vacuum.

Of course, it is then necessary to have some sort of means to hold the cement in the mixing chamber during mixing and prevent it falling down into the syringe, but then to allow the mixed cement to fall into the syringe.

A solution to these problems was found according to a further aspect of the present invention which provides an orthopaedic cement mixing apparatus comprising a mixing chamber and a dispensing chamber, the mixing chamber having an outlet aperture and the dispensing chamber having an inlet aperture, the inlet aperture and the outlet aperture being in cement flow communication, and further comprising closure means for separating the inlet aperture and the outlet aperture to prevent the flow of cement from the mixing chamber to the dispensing chamber, wherein the closure means is moveable between a closed position whereby flow of cement from the mixing chamber to the dispensing chamber is prevented and an open position whereby cement can flow from the mixing chamber to the dispensing chamber.

In the currently preferred embodiment, therefore, the closure is a tap defining a channel or passage therethrough, which fits between the mixing chamber and the dispensing chamber. During mixing, the channel or passage is located out of communication with the outlet aperture of the mixing chamber and the inlet aperture of the dispensing chamber. After mixing, the tap is rotated by a switch or handle extending to the outside of the apparatus so that the channel or passage is aligned with the outlet of the mixing chamber and the inlet of the dispensing chamber so that the cement can then fall from the mixing chamber through that channel into the dispenser.

In the present invention, instead of a tap, a ball with a holder through is used as the closure, again located between the mixing chamber and the dispensing chamber. During mixing, the channel or passage is located out of communication with the outlet aperture of the mixing chamber and/or the inlet aperture of the dispensing chamber. After mixing, the ball is rotated by a handle so that the hole is aligned with the outlet of the mixing chamber and the inlet of the dispensing chamber allowing cement to fall through from the mixing chamber to the dispensing chamber.

Again, it is envisaged that these closure arrangements could be incorporated into any mix-and-dump system and provide advantages.

With such an arrangement, however, the vacuum switching mechanism described at the beginning is not really ideal since, during mixing, air could come into the mixing chamber from the syringe, if the ball is not completely sealed. Thus, with this arrangement, ideally a vacuum would need to be applied to the whole device for the whole time, i.e. to the mixing chamber and to the syringe both during mixing and dumping.

Thus, the preferred device combines the off-centre mixing arrangement of the earlier Summit bowl described in WO 93/10892, together with the counter-rotating paddle and scraper and has the ball closure and has vacuum applied both to the mixing chamber and the syringe.

Preferred embodiments of the invention will now be described, by way of example only, with reference to the accompanying drawings.
Figure 1 shows a first embodiment of a mixing apparatus according to the present invention;
Figure 2 shows the piston closure arrangement of an embodiment of the present invention;
Figure 3 shows an alternative piston arrangement to that of Figure 2;
Figure 4 shows yet a further piston arrangement;
Figure 5 shows a cross-section of a further embodiment of the device with a different closure arrangement;
Figures 6A and 6B show views of an embodiment of a closure; and
Figures 6C and 6D show the closure of Figures 6A and 6B located in a mixer respectively in its closed and open positions;
Figure 7 shows a cross-section of a mixer with the tap closure in an open position;
Figure 8 shows a cross-section of a mixer with the tap closure in a closed position;
Figure 9 shows a collar for housing a closure;
Figure 10 shows a mixing chamber adapted for use with the collar of Figure 9;
Figures 11A and 11B show another embodiment of a mixer with the closure in open and closed positions respectively; and

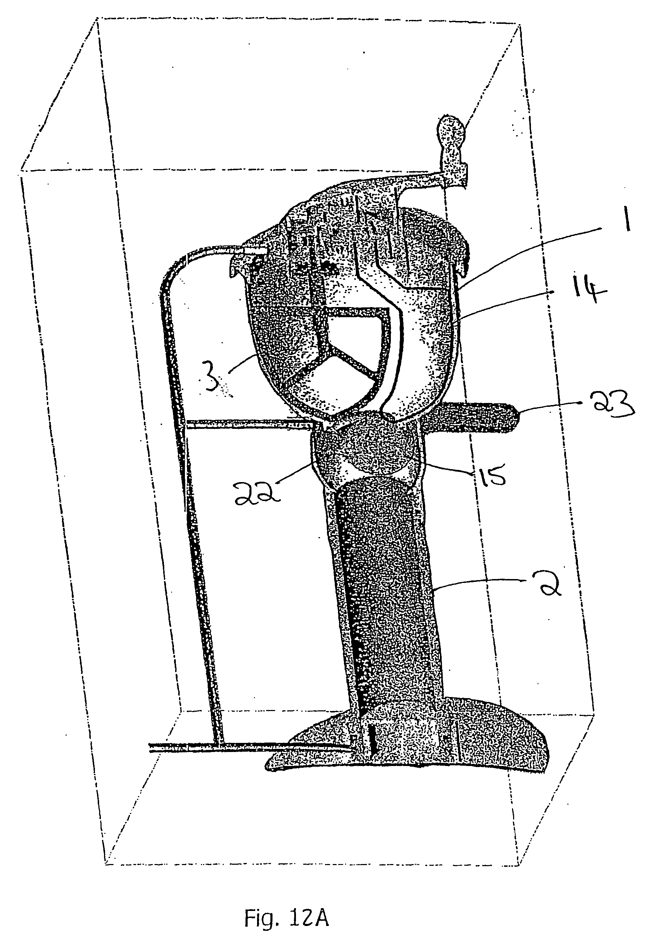

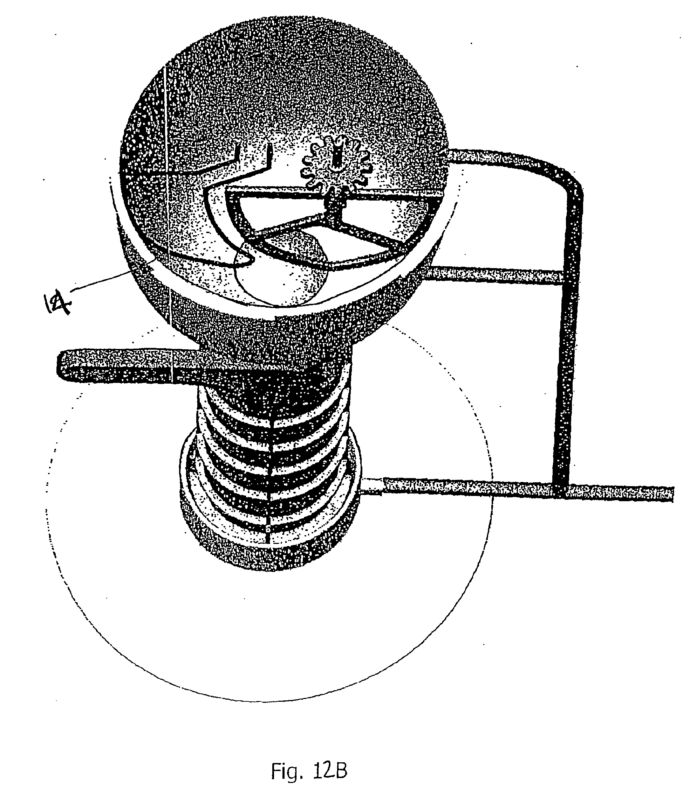

Figures 12A and 12B show, respectively, a sectional and a top perspective view of a mixer according to a preferred embodiment.

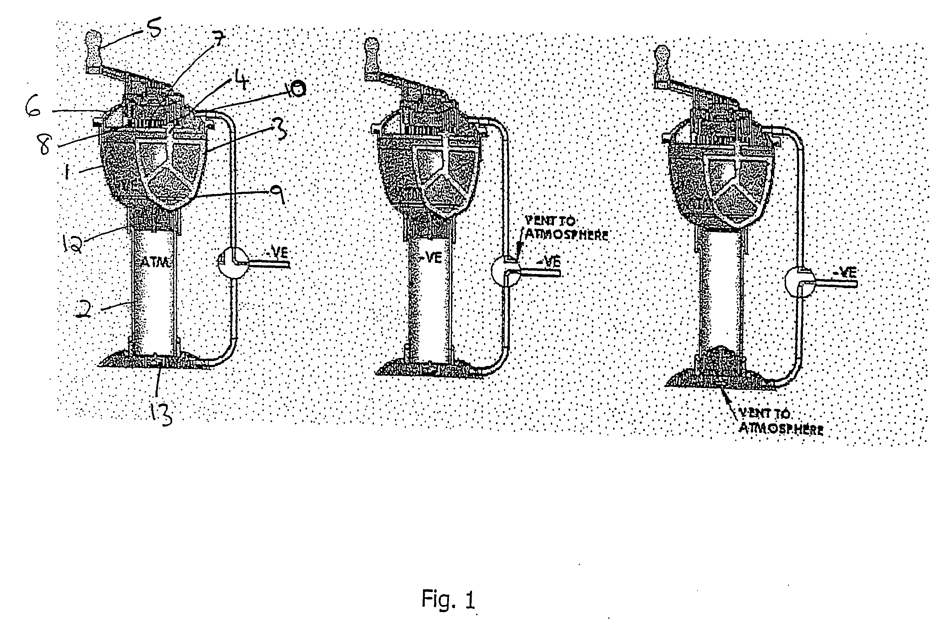

Referring first to Figure 1, the mixing apparatus comprises a first bowl-shaped mixing chamber 1 and a second, cylindrical mixing chamber 2. A mixing paddle 3 extends through the mixing chamber comprising at least one blade supported by a rotatable shaft 4. The shaft, and, therefore, the mixing paddle, is rotated by means of a handle 5. The handle is mounted in a lid 6 adapted to be sealingly fitted onto the top of the funnel 1. A gear mechanism 7 is provided to cause rotation of the shaft 4 about its own axis, as well as rotation of the shaft axis around the mixing chambers.

The preferred gear mechanism comprises a fixed, circular, toothed rack 8 arranged coaxially with respect to the rotation axis of the handle 5, and provided on the underside of the lid 6. The mixing paddle 3 comprises radially extending mixing blades 9, mounted on the shaft 4. The shaft 4 is rotatably mounted, at one end, into the handle 5. A cog-wheel 10 is fixedly attached to the upper part of the shaft 4 for intermeshing engagement with the toothed rack 8.

The lid is preferably also provided with a vacuum port (not shown) for connection to a vacuum pump (not shown). The lid 6 is preferably provided with a seal 11 for sealing between the lid 6 and the rim of the mixing chamber 1. Locking means (not shown) may also be provided between the lid 6 and the mixing chamber 4 and these means will be discussed further below.

The mixing chamber 1 is fitted over one end of the mixing cylinder 2 in a sealing engagement. The connection may be by means of a pushfit or a screw thread, for example.

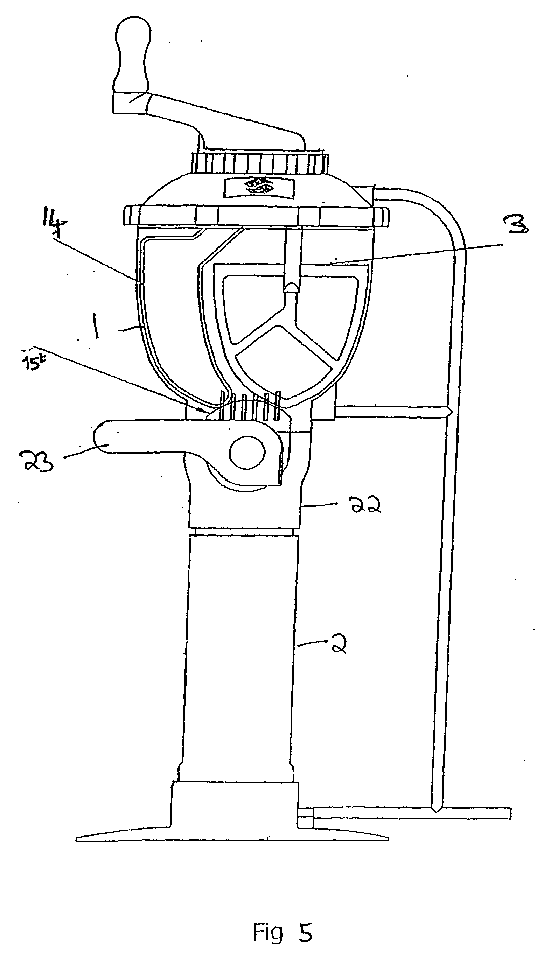

In the embodiment shown, a plunger 12 for ejecting the mixed cement is slidingly located between the mixing chamber and 2. In an alternative embodiment described in relation to Figures 5, 6 and 7, the plunger 12 is slidingly located at the other end of the cylinder 2. This 'other' end of the cylinder 2 is adapted to be received in a stand 13 and may be secured to the stand by corresponding screw threads. A seal may also be provided between the cylinder body 1 and the stand 13.

Vacuum is applied to the mixing chamber via a port in the lid and is applied to the dispensing syringe via a port in the stand. A switching arrangement is provided in the vacuum line to allow the vacuum to be selectively applied to the mixing chamber and to the dispensing chamber or syringe.

The method of use of the mixing apparatus will now be described.

The mixing apparatus is provided to the user in assembled form, as shown in Fig. 1.

In one embodiment, the apparatus is provided as a pre-filled device, i.e. the cement is already provided in the mixer, e.g. in a closed bag or some other retaining means.

In the case of the pre-filled container, the closed bag or retaining means is removed, leaving the cement in the chamber.

Alternatively, where the mixer is not pre-filled, the lid 6 and attached mixing arrangement is removed, and the cement powder is inserted into the mixing chamber.

The bowl shape allows a greater volume of dry cement powder to be accommodated and the wider top part makes the introduction of cement without spillage, easier.

The monomer ampoule is then broken and added to the cement in the mixing chamber and the lid is replaced in sealing engagement with the open mouth of the chamber.

Where the mixing is carried out under vacuum, the vacuum port is connected via a length of PVC tubing (not shown) to the vacuum pump to create a vacuum. In this first embodiment, the vacuum switch is switched to apply vacuum to the mixing chamber, whilst the dispensing chamber is at atmospheric pressure. In other embodiments vacuum is applied to both chambers. Mixing of the cement components is then carried out by rotation of the handle 5 by the user. The complete mixing apparatus may be held in the hand or may be placed in the base 13 and supported on a flat surface such as a table.

Wastage is reduced and the mixing effect is improved by the additional scraper paddle 14 shown most clearly in Figs. 14A and 14B. The scraper paddle should have a relatively thin profile to cut through the cement but is shaped in the form of a paddle so as to assist in mixing, and, further, to extend out to the inner wall of the bowl to scrape cement from the bowl surface. The scraper paddle is attached to the gear mechanism 7 such that as the handle 5 is rotated, during mixing, the scraper paddle rotates around the mixing bowl in counter-rotation with the mixing paddle shaft 4. Thus, the scraper paddle is shaped so as to have a radially outer curve which essentially follows the shape of the inside surface of the bowl 1 and a radially inner profile which is curved so as to pass by the paddle 3 during rotation.

The profile of the mixing blade 9 in the mixing chamber 1 is such that it will push the cement powder down towards the bottom of the mixing chamber, as the components are mixed. As the two components mix, the volume of cement reduces and when completely mixed, the cement will be capable of being accommodated within the cylindrical chamber 2.

With the rotating axis arrangement, the operator rotates the handle 5 which causes planetary movement of the shaft 4 about its central axis and, at the same time, causes the cog-wheel 10 to mesh with the rack 8 so as to drive the cog-wheel, producing rotation of a paddle about the axis of the shaft. Thus, due to the gear mechanism provided by the toothed rack and the cog-wheel, rotation of the handle causes the paddle to move around the mixing chamber in planetary fashion and, at the same time, to rotate about its own axis.

Such a mechanism enables the paddle to rotate several times for each turn of the handle and results in a more than 90% coverage of the mixing chamber area. Preferably, one rotation of the handle does not cause a whole number of rotations of the paddle, so the paddle is in a different orientation at the beginning and end of a particular cycle of the axial movement. This helps to avoid dead spots being formed in the cement and improves mixing. In the preferred embodiment, this is further improved by the scraper paddle.

Once the cement is thoroughly mixed (the surgeon can visually monitor this by viewing through the transparent walls of the container or through a viewing window provided in the container, and an experienced surgeon will also be able to tell by the resistance of the cement to mixing, whether the cement is ready), it is "dumped" into the dispensing chamber or syringe body. In the embodiment shown in Figure 1, this is done by switching the vacuum from the mixing chamber 1 to the dispensing chamber 2. The mixing chamber is then at atmospheric pressure and the pressure differential is sufficient to draw the piston or plunger 12 between the mixing chamber and the dispensing chamber, and the mixed cement down into the body of the dispensing chamber until the piston or plunger reaches its end position at the bottom of the dispensing chamber. In other embodiments, as will be described in relation to the other figures, a closure 15 between the mixing chamber 1 and the dispensing chamber 2 will be removed or opened providing a passage from the outlet 16 of the mixing chamber through the inlet 17 of the dispensing chamber through which the mixed cement falls. Once the mixed cement has all been 'dumped' into the syringe body, the mixing chamber 1, the lid 6 and the mixing paddle 3 are removed from the cylindrical mixing chamber 2 and are replaced by a dispensing nozzle.

Whilst all of these parts can be removed individually, in the preferred embodiment, the lid, to which the mixing mechanism is already attached, is provided with a locking arrangement to lock it to the mixing chamber part 1, so that these parts can all be removed together. This reduces the risk of any remaining unmixed cement powder in the top part of the mixing chamber 1 falling into the mixed cement. A blade wiping slot may be introduced between the mixing bowl and the syringe, through which the blade is drawn as it is removed, to ensure that all the mixed cement remains in the cylindrical chamber 2.

An applicator nozzle (not shown) is then attached to the open end of the cylindrical chamber 2, from which the mixing chamber 1 has been removed. The cylindrical chamber 2 is also removed from the base 13, if the base has been used. The mixed cement is then forced through the nozzle under the action of the plunger 12, to be applied to the appropriate site. Different types of plunger may be used to force the cement out through the nozzle, for example a hand-operated gun may be used or a gas powered pressure gun. The dispensing mechanism may be as described in, for example, EP-A-0744991.

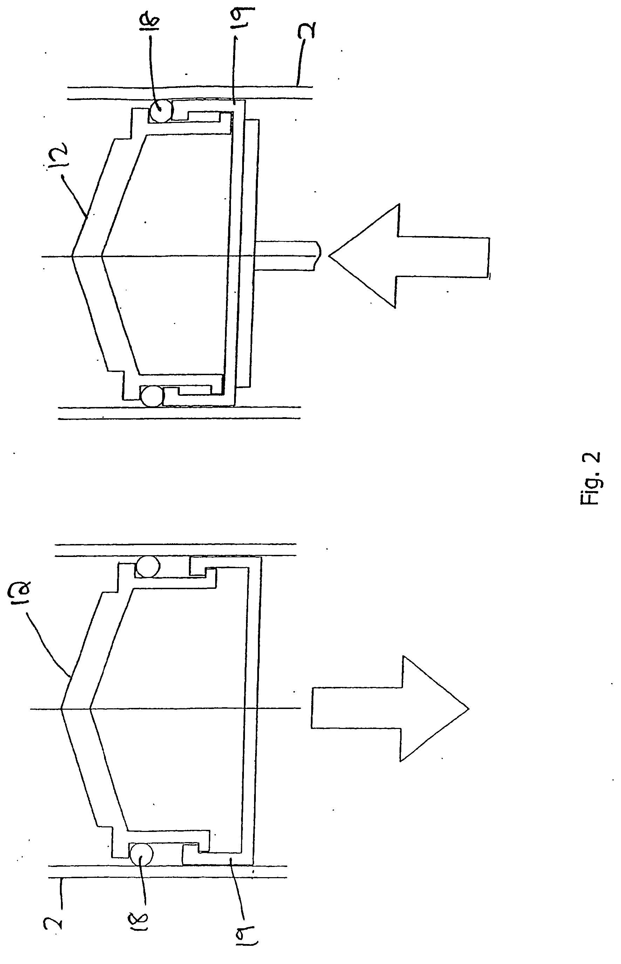

Figures 2, 3 and 4 show different piston arrangements which can be used where the piston 12 provides the closure between the mixing chamber 1 and the dispensing chamber 2 during mixing and whereby that piston must, therefore, be held in place, in a first, closure position during mixing. After mixing, the piston 12 then slides down the body of the dispensing chamber until it is located at the bottom of that chamber to act as a piston for ejecting the mixed cement.

In the embodiment shown in Figure 2, the closure or piston is provided with an O-ring 18 and a collar 19 arrangement as shown in Figure 2A. In the first, closure; position, the O-ring 18 is adapted to operate light radial pressure to seal against the vacuum in the mixing chamber. The piston is then light enough to fall down to the bottom of the chamber with the mixed cement. However, such a light piston arrangement is not sufficient to force out the mixed cement and, therefore, this embodiment is provided with the collar 19 component which, when the piston reaches its bottom position, adds to squash against the O-ring causing a greater radial pressure of the O-ring, outwards, forcing the seal into a tighter sealing engagement with the inner walls of the syringe body providing a stronger piston unit to push against the cement.

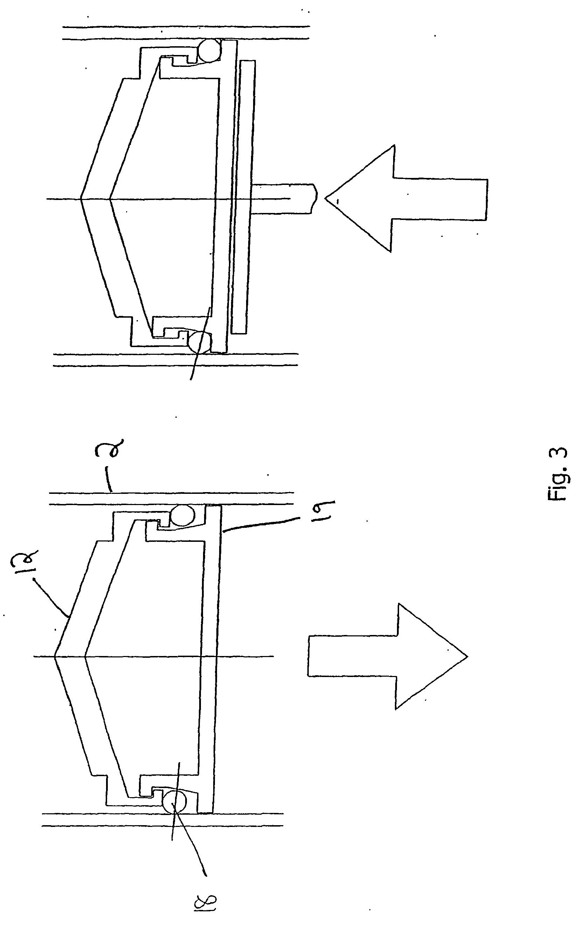

The embodiment shown in Figure 3 is similar to that of Figure 2, but the engagement of the collar 19 and the piston 12 is slightly different.

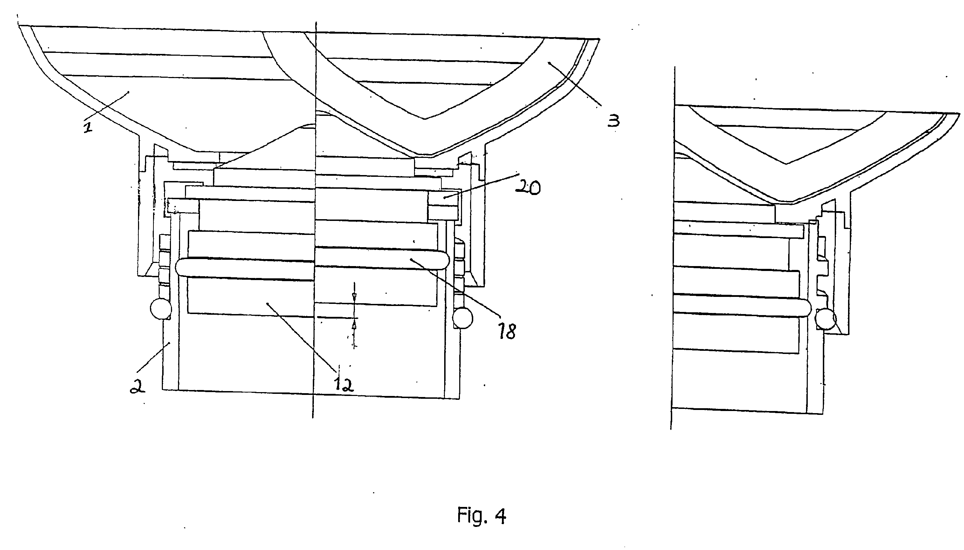

In Figure 4, a spacer 20 (which can be seen more clearly in Figure 5) is provided to hold the piston 12 in place during transit. When the apparatus is ready to be used, the user removes the spacer and the piston is then forced upwards sealing against the bottom of the mixing chamber. The O-ring 18 stiction is relieved against the syringe. After mixing, the syringe then falls due to the vacuum switching as described above.

In a further embodiment, as described above, the piston is not located at the top of the syringe body during mixing and does not provide the closure between the mixing chamber and the syringe body. Instead, a separate closure 15 is provided between the mixing chamber and the syringe body and the piston 12 itself is located, during mixing, at the bottom of the syringe body.

Fig. 5 shows a preferred closure arrangement comprising a rotatable tap member 15^{t} and means for rotating the tap between a first, closed position where the tap covers the mixing chamber outlet and/or dispensing chamber inlet, and in which cement cannot flow from the mixing chamber through the syringe and a second position in which a passage is aligned with the outlet aperture of the mixing chamber and the inlet aperture of the dispensing chamber to provide a passage for the cement to flow from the mixing chamber to the dispensing chamber.

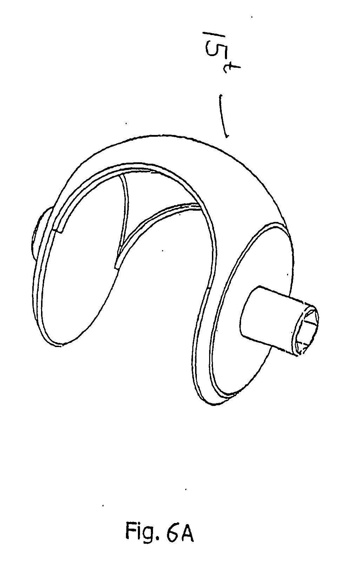

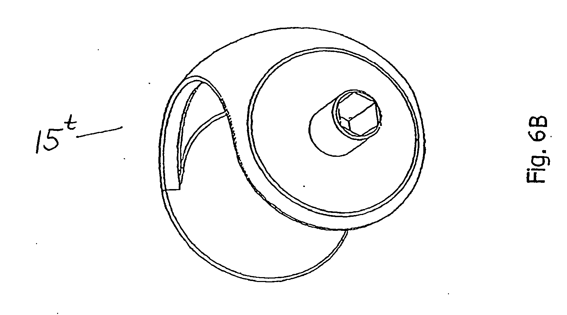

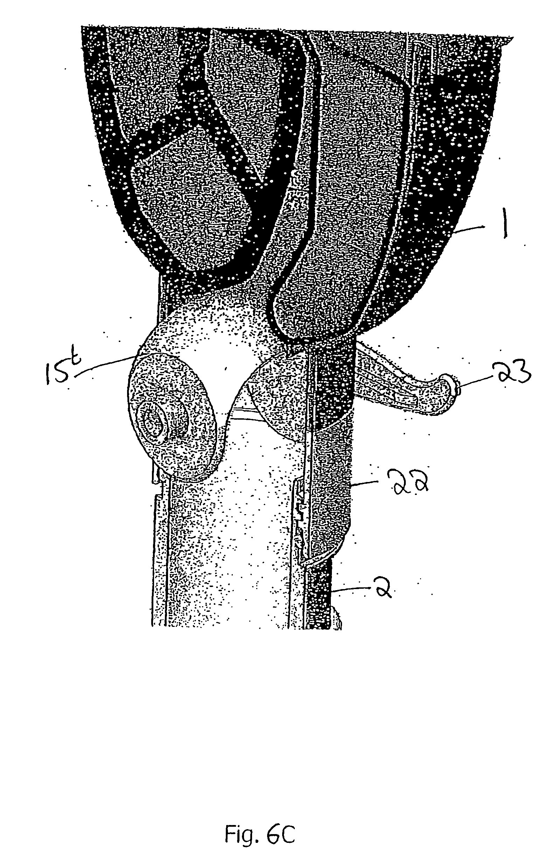

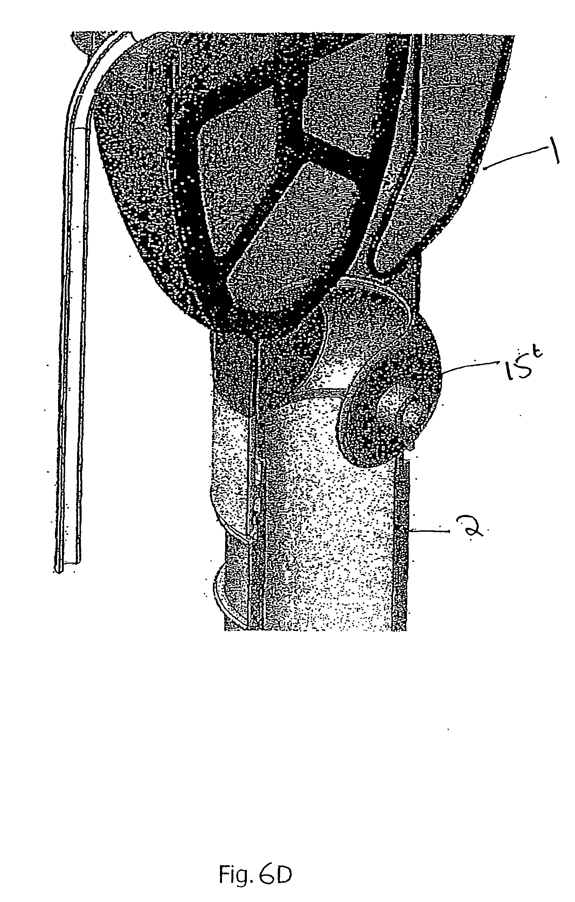

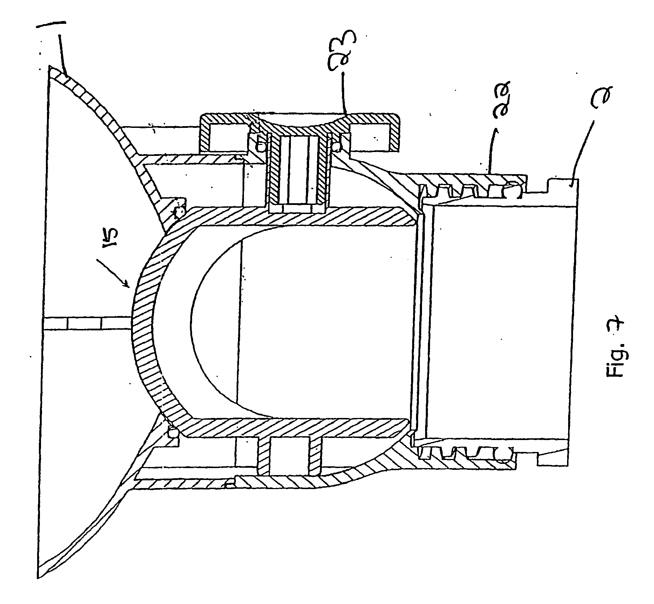

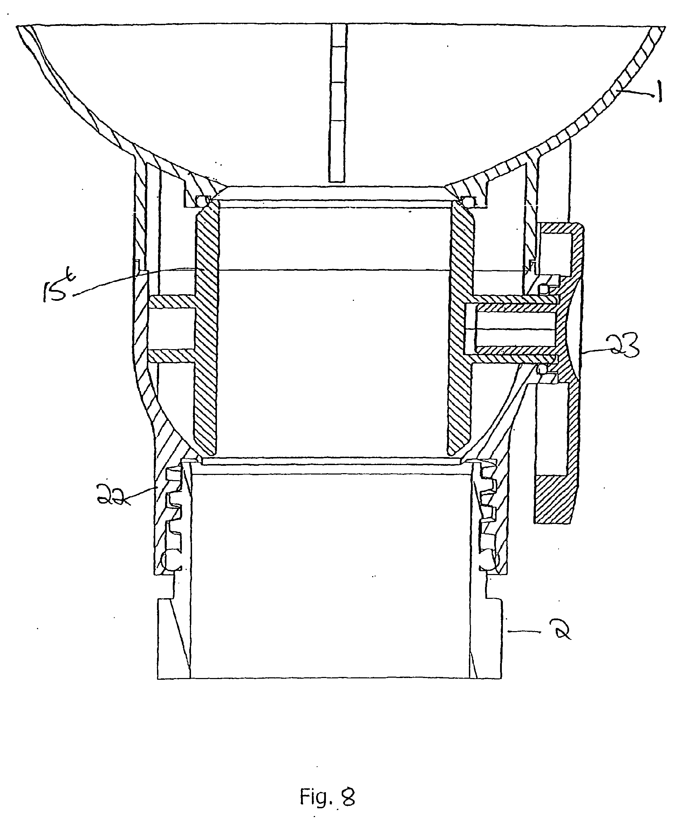

In the preferred embodiment, as shown in Fig. 5, the tap arrangement is mounted in a collar or housing which fits between the mixing chamber and the dispensing chamber. Fig. 5 shows the tap 15^{t} and its housing 22 in position between the mixing chamber and the dispensing chamber. As can be seen, a handle or switch 23 is provided, attached to the tap, and extending through the housing or collar to be operated by the user. The actual tap member itself is shown in Figs. 6A, 6B and, in position, in Figs. 6C and 6D and is, essentially, a C-shaped component rotatable about an axis. Fig. 7 shows the tap member 15^{t} in its closed position, whereby the curve of the C-shape provides a closure across the mixing chamber outlet aperture to prevent cement exiting through that aperture. After mixing, the switch or handle 22 is rotated by the user to the open position as shown in Fig. 8, whereby the curve of the C rotates out of engagement with the aperture and the C-shape then defines a channel or passage between the outlet aperture of the mixing chamber and the inlet aperture of the dispensing chamber.

Preferably, seals 24 are provided around the outlet aperture of the mixing chamber and where the switch or handle passes through the wall of the collar or housing of the tap member.

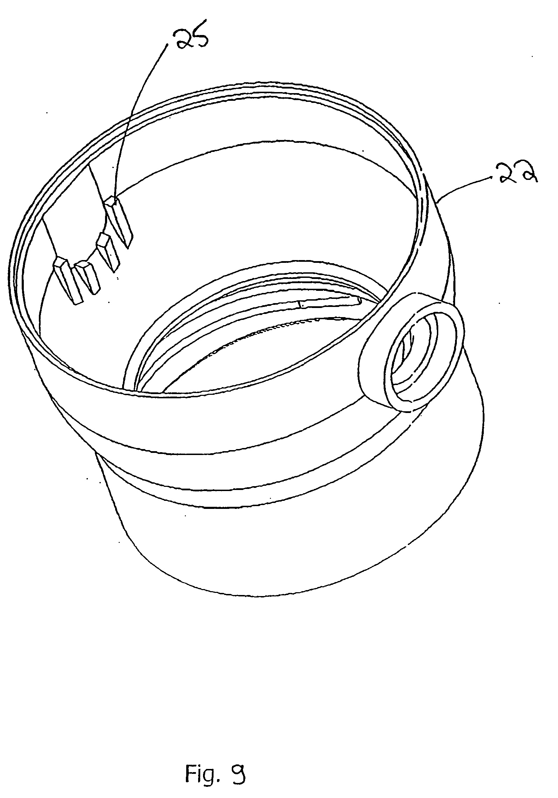

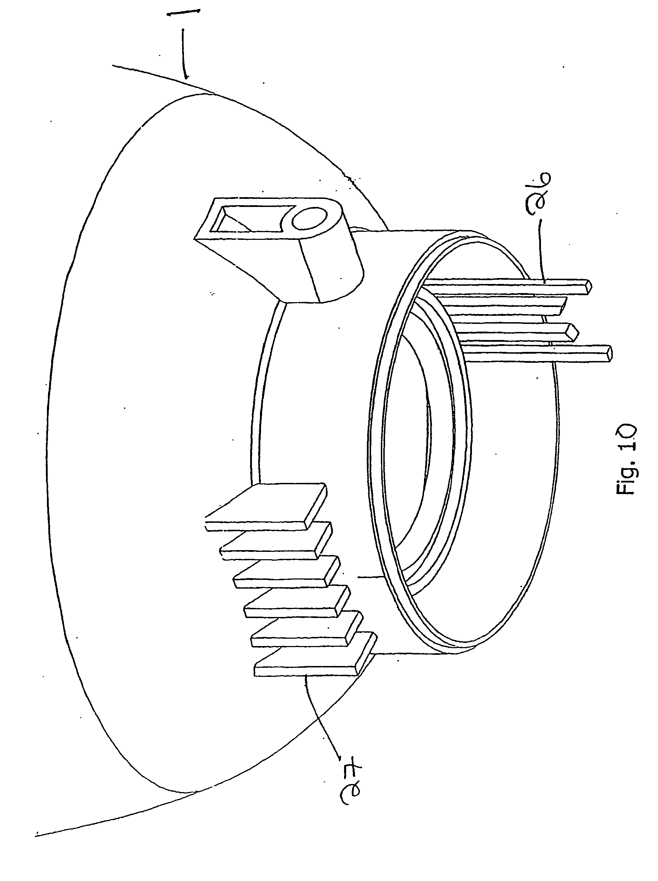

Fig. 9 shows a schematic view of the collar 22 or housing of the tap member. Ribs or grooves 25 may be provided for locating the axis of the tap member.

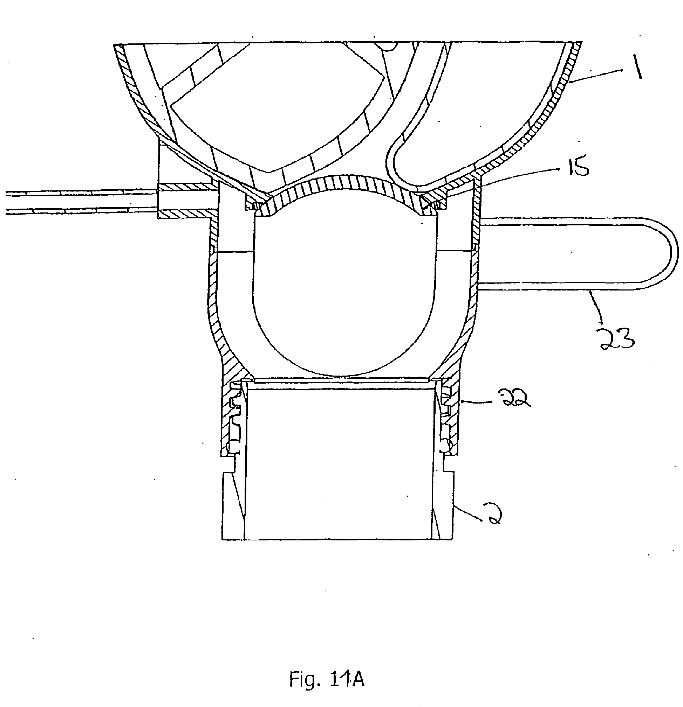

Fig. 10 shows the bottom of the mixing chamber in a preferred embodiment. Again, locating ribs or grooves 26 are provided for engagement with the locating grooves of the collar or housing of the tap member. A switch stop 27 may be provided to prevent over-rotation of the switch or handle beyond the 90° rotation required to move the tap from its open to its closed position.

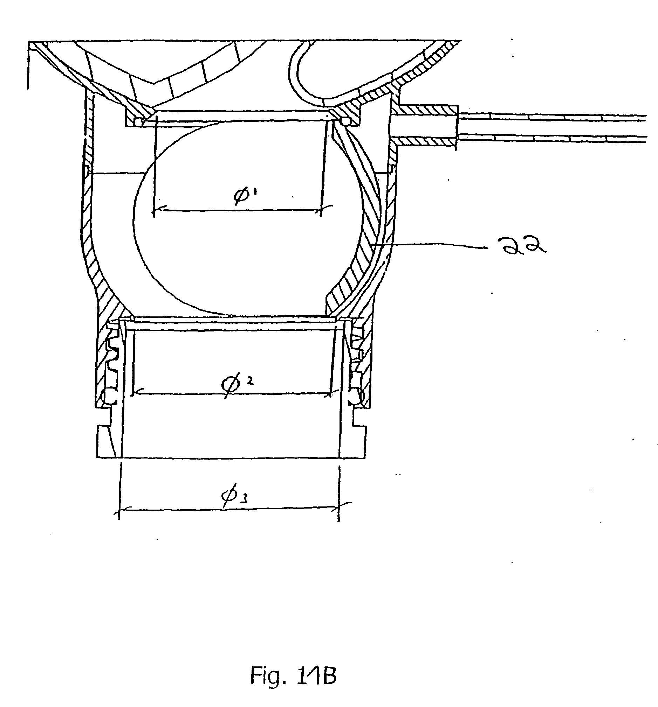

As shown in Figs. 11A and 11B, different dimensions may be provided such that, for example, the outlet aperture 16 of the mixing chamber is smaller than the diameter of the passageway formed by the tap and the inlet aperture 17 of the dispensing chamber. This allows cement to be dumped from the mixing chamber to the dispensing chamber through the passage without too much cement touching the walls of the tap mechanism and the top of the syringe, thus reducing wastage and preventing cement adversely affecting the tap mechanism.

Figs. 11A and 11B show schematic views of the tap in its open and closed positions, respectively.

In this arrangement, it is preferred that a vacuum is applied to the mixing chamber and to the dispensing chamber and syringe base during both mixing and dumping.

The fact that the bowl-shaped mixing chamber 1 is used in combination with the syringe chamber 2, allows larger quantities of cement to be mixed, and the cylindrical mixing chamber 2, which forms the body of the dispensing syringe, need only be large enough to accommodate the mixed cement volume. This can, when cements such as Simplex are used, be considerably less than the unmixed volume.

It is envisaged that different syringe sizes can be used in this invention, according to the different volumes and types of cement being used. This provides greater flexibility to the user and optimises the syringe body size. Essentially, using the present invention, the shortest possible syringe body size can be used for the maximum desired quantity of mixed cement. It is envisaged that existing syringe bodies could be used with the present invention.

The present arrangement thus results in a mixing apparatus in which large quantities of cement can be efficiently and thoroughly mixed and dispensed. The system is easy to use, and uses a familiar mixing motion, popular with users.

## Claims

1. An orthopaedic cement mixing apparatus comprising a mixing chamber (1) and a dispensing chamber (2), the mixing chamber (1) having an outlet aperture and the dispensing chamber having an inlet aperture (17), the inlet aperture and the outlet aperture being in cement flow communication, and **characterised by** further comprising closure means for separating the inlet aperture and the outlet aperture to prevent the flow of cement from the mixing chamber to the dispensing chamber, wherein the closure means is moveable between a closed position whereby flow of cement from the mixing chamber to the dispensing chamber is prevented and an open position whereby cement can flow from the mixing chamber to the dispensing chamber; and wherein the closure comprises a ball (15t) with a hole therethrough, located between the mixing chamber and the dispensing chamber.

2. The orthopaedic cement mixing apparatus of claim 1, wherein a vacuum is applied to the mixing chamber and to the dispensing chamber both during mixing and dumping.

3. The orthopaedic cement mixing apparatus of claims 1 or 2, comprising a cylindrical dispensing chamber, further comprising means for applying a vacuum to the mixing chamber (1) and to the dispensing chamber (2); and wherein the apparatus further comprises switching means for switching the applied vacuum between the mixing chamber (1) and the dispensing chamber (2).

4. The orthopaedic cement mixing apparatus as claimed in claim 2 or 3, wherein the dispensing chamber is a cylindrical body of a dispensing syringe.

5. The orthopaedic cement mixing apparatus of any of claims 1 to 4, further comprising a mixing paddle (3) extending into said mixing chamber (1) and a rotatable handle coupled to said paddle by a gear mechanism arranged such that rotation of said handle causes said paddle to rotate about its own axis and also moves the axis of rotation of the paddle within the chamber whereby the paddle is moved around substantially the entire cement containing region of the interior of the chamber.

6. The orthopaedic cement mixing apparatus of any preceding claim, wherein the mixing chamber is a bowl.

7. The orthopaedic cement mixing apparatus of claim 5, further comprising a scraper element connected to said gear mechanism so as to rotate with the axis of rotation of the paddle in the same or in the opposite direction.

8. The orthopaedic cement mixing apparatus of claim 7, wherein the scraper is shaped such that it extends radially from the gear mechanism to the inside wall of the mixing chamber and extends axially to conform to the shape of the inside wall of the mixing chamber and extends axially to conform to the shape of the inside wall of the mixing chamber.

9. The orthopaedic cement mixing apparatus of any preceding claim, wherein in a first position the closure means separates the outlet aperture from the inlet aperture preventing flow of cement from the mixing chamber to the dispensing chamber; **characterized in that** the closure means is held in its first position by a releasable fastening means.

10. The orthopaedic cement mixing apparatus as claimed in claim 9, wherein the releasable fastening means comprises a removable pin which passes through the wall of the dispensing chamber and into the closure.

11. The orthopaedic cement mixing apparatus as claimed in claim 9, wherein the releasable fastening means comprises an O-ring which is dimensioned such that it provides a friction grip between the closure wall and the inner wall of the dispensing chamber.

12. The orthopaedic cement mixing apparatus as claimed in claim 1 or 11, wherein the closure means is provided with a light seal or an O-ring (18) sufficient to hold the closure means in place between the mixing chamber (3) and the dispensing chamber (2).

## Patentansprüche

1. Vorrichtung zum Mischen von orthopädischem Zement, mit einer Mischkammer (1) und einer Abgabekammer (2), wobei die Mischkammer (1) eine Auslassöffnung und die Abgabekammer eine Einlassöffnung (17) aufweist, wobei die Einlassöffnung und die Auslassöffnung in Zementfluss-Kommunikation sind, **dadurch gekennzeichnet, dass** sie ferner Schließmittel bzw. Verschlussmittel zum Trennen bzw. Separieren der Einlassöffnung und der Auslassöffnung zur Verhinderung eines Flusses von Zement von der Mischkammer in die Abgabekammer aufweist, wobei die Verschlussmittel bewegbar sind zwischen einer geschlossenen Position, wodurch ein Fluss von Zement von der Mischkammer in die Abgabekammer verhindert wird, und einer offenen Position, wodurch Zement von der Mischkammer in die Abgabekammer fließen kann; und wobei der Verschluss einen Ball bzw. eine Kugel (15t) mit einem durch sie hindurch verlaufenden Loch aufweist, welche zwischen der Mischkammer und der Abgabekammer angeordnet ist.

2. Vorrichtung zum Mischen von orthopädischem Zement nach Anspruch 1, bei der während des Mischens und des Ablassens bzw. des Dumpings die Mischkammer und die Abgabekammer mit einem Vakuum bzw. einem Unterdruck beaufschlagt werden.

3. Vorrichtung zum Mischen von orthopädischem Zement nach einem der Ansprüche 1 oder 2, mit einer zylindrischen Abgabekammer, ferner mit Mitteln zum Beaufschlagen der Mischkammer (1) und der Abgabekammer (2) mit einem Vakuum; und wobei die Vorrichtung ferner Schaltmittel aufweist, um das anliegende bzw. aufgebrachte Vakuum zwischen der Mischkammer (1) und der Abgabekammer (2) hin- und her zu schalten.

4. Vorrichtung zum Mischen von orthopädischem Zement nach einem der Ansprüche 2 oder 3, bei der die Abgabekammer ein zylindrischer Körper einer Abgabespritze ist.

5. Vorrichtung zum Mischen von orthopädischem Zement nach einem der Ansprüche 1 bis 4, ferner mit einem Mischpaddel (3), das sich in die Mischkammer (1) hinein erstreckt, und einem drehbaren Griff, der mittels eines Getriebemechanismus mit dem Paddel gekoppelt bzw. verbunden ist, der ausgebildet ist, dass eine Drehung des Griffes eine Drehung des Paddels um seine eigene Achse bewirkt, und auch die Drehachse des Paddels innerhalb der Kammer bewegt, wodurch das Paddel im wesentlichen innerhalb des gesamten Zement enthaltenden Bereiches des Inneren der Kammer bewegt wird.

6. Vorrichtung zum Mischen von orthopädischem Zement nach einem der vorstehenden Ansprüche, bei der die Mischkammer eine Schüssel ist.

7. Vorrichtung zum Mischen von orthopädischem Zement nach Anspruch 5, ferner mit einem Kratzerelement, das mit dem Getriebemechanismus verbunden ist, um mit der Drehachse des Paddels in der gleichen oder in der entgegengesetzten Richtung zu rotieren bzw. sich zu drehen.

8. Vorrichtung zum Mischen von orthopädischem Zement nach Anspruch 7, bei der der Kratzer derart geformt ist, dass er sich radial von dem Getriebemechanismus zu der Innenwand der Mischkammer erstreckt, und sich axial erstreckt, um mit der Form der Innenwand der Mischkammer übereinzustimmen, und sich axial erstreckt um mit der Form der Innenwand der Mischkammer überein zu stimmen.

9. Vorrichtung zum Mischen von orthopädischem Zement nach einem der vorstehenden Ansprüche, bei der in einer ersten Position die Verschlussmittel die Auslassöffnung von der Einlassöffnung trennen, und einen Fluss von Zement von der Mischkammer in die Abgabekammer verhindern, **dadurch gekennzeichnet, dass** die Verschlussmittel durch lösbare Befestigungsmittel in ihrer ersten Position gehalten werden.

10. Vorrichtung zum Mischen von orthopädischem Zement nach Anspruch 9, bei der die lösbaren Befestigungsmittel einen entfernbaren Stift umfassen, der sich durch die Wand der Abgabekammer und in den Verschluss hinein erstreckt.

11. Vorrichtung zum Mischen von orthopädischem Zement nach Anspruch 9, bei der die lösbaren Befestigungsmittel einen O-Ring aufweisen, der derart dimensioniert ist, dass er einen Reibhalt zwischen der Verschlusswand und der Innenwand der Abgabekammer zur Verfügung stellt.

12. Vorrichtung nach einem der Ansprüche 1 oder 11, bei der die Verschlussmittel mit einer leichten Dichtung bzw. einer Lichtdichtung oder einem O-Ring (18) ausgebildet sind, die bzw. der ausreicht, um die Verschlussmittel an ihrer Position zwischen der Mischkammer (3) und der Abgabekammer (2) zu halten.

## Revendications

1. Dispositif de mélange de ciment orthopédique comportant une chambre de mélange (1) et une chambre de distribution (2), la chambre de mélange (1) ayant une ouverture de sortie et la chambre de distribution ayant une ouverture d'entrée (17), l'ouverture d'entrée et l'ouverture de sortie étant en communication d'écoulement de ciment, et **caractérisé en ce qu'**il comporte en outre des moyens de fermeture pour séparer l'ouverture d'entrée et l'ouverture de sortie afin d'empêcher l'écoulement de ciment de la chambre de mélange vers la chambre de distribution, dans lequel les moyens de fermeture sont mobiles entre une position fermée dans laquelle l'écoulement de ciment de la chambre de mélange vers la chambre de distribution est empêché, et une position ouverte, dans laquelle le ciment peut s'écouler de la chambre de mélange vers la chambre de distribution ; et dans lequel la fermeture comporte une bille (15t) avec un trou à travers celle-ci, située entre la chambre de mélange et la chambre de distribution.

2. Dispositif de mélange de ciment orthopédique selon la revendication 1, dans lequel un vide est appliqué à la chambre de mélange et à la chambre de distribution pendant le mélange et le déversement.

3. Dispositif de mélange de ciment orthopédique selon les revendications 1 ou 2, comportant une chambre de distribution cylindrique, comportant en outre des moyens d'appliquer un vide à la chambre de mélange (1) et à la chambre de distribution (2) ; et dans lequel le dispositif comporte en outre des moyens de commutation pour commuter le vide appliqué entre la chambre de mélange (1) et la chambre de distribution (2).

4. Dispositif de mélange de ciment orthopédique selon la revendication 2 ou 3, dans lequel la chambre de distribution est un corps cylindrique d'une seringue de distribution

5. Dispositif de mélange de ciment orthopédique selon l'une quelconque des revendications 1 à 4, comportant en outre une palette de mélange (3) s'étendant dans ladite chambre de mélange (1) et une poignée rotative couplée à ladite palette par un mécanisme d'engrenage agencé de sorte qu'une rotation de ladite poignée fait tourner ladite palette autour de son propre axe et déplace également l'axe de rotation de la palette dans la chambre, de sorte que la palette est déplacée autour de sensiblement toute la zone contenant du ciment de la partie intérieure de la chambre.

6. Dispositif de mélange de ciment orthopédique selon l'une quelconque des revendications précédentes, dans lequel la chambre de mélange est une cuvette.

7. Dispositif de mélange de ciment orthopédique selon la revendication 5, comportant en outre un élément racleur relié audit mécanisme d'engrenage afin de tourner avec l'axe de rotation de la palette dans la même direction, ou dans la direction opposée.

8. Dispositif de mélange de ciment orthopédique selon la revendication 7, dans lequel le racleur est mis en forme de sorte qu'il s'étend radialement à partir du mécanisme d'engrenage vers la paroi intérieure de la chambre de mélange, et s'étend axialement pour se conformer à la forme de la paroi intérieure de la chambre de mélange, et s'étend axialement pour se conformer à la forme de la paroi intérieure de la chambre de mélange.

9. Dispositif de mélange de ciment orthopédique selon l'une quelconque des revendications précédentes dans lequel, dans une première position, les moyens de fermeture séparent l'ouverture de sortie de l'ouverture d'entrée en empêchant un écoulement de ciment de la chambre de mélange vers la chambre de distribution ; **caractérisé en ce que** les moyens de fermeture sont retenus dans leur première position par des moyens de fixation libérables.

10. Dispositif de mélange de ciment orthopédique selon la revendication 9, dans lequel les moyens de fixation libérables comportent une broche amovible qui passe à travers la paroi de la chambre de distribution et dans la fermeture.

11. Dispositif de mélange de ciment orthopédique selon la revendication 9, dans lequel les moyens de fixation libérables comportent un joint torique qui est dimensionné de sorte qu'il fournit une préhension à frottement entre la paroi de fermeture et la paroi intérieure de la chambre de distribution.

12. Dispositif de mélange de ciment orthopédique selon la revendication 1 ou 11, dans lequel les moyens de fermeture sont munis d'un joint d'étanchéité léger ou d'un joint torique (18) suffisant pour retenir les moyens de fermeture en place entre la chambre de mélange (3) et la chambre de distribution (2).
